# FASCICULE DE BREVET EUROPEEN

(11) **EP 1 631 219 B1**
(45) Date de publication et mention de la délivrance du brevet: **03.01.2007**
(21) Numéro de dépôt: 04767276.1
(22) Date de dépôt: 07.06.2004
(51) Int. Cl.: A61F 2/34

(54) **INSERT POUR CUPULE D IMPLANT COTYLO DIEN POUR PROTHESE ARTICULAIRE, IMPLANT COTYLO DIEN ET PROTHESE ARTICULAIRE**
EINSATZ FÜR EINE COTYLOID-IMPLANTATPFANNE FÜR EINE GELENKPROTHESE, COTYLOID-IMPLANTAT UND GELENKPROTHESE
INSERT FOR A COTYLOID IMPLANT CUP FOR A JOINT PROSTHESIS, COTYLOID IMPLANT AND JOINT PROSTHESIS

(30) Priorité: 06.06.2003 FR 0306893
(43) Date de publication de la demande: 08.03.2006
(73) Titulaire: Biotechni, 13600 La Ciotat (FR)
(72) Inventeur: CALAMEL, Serge, F-13600 La Ciotat (FR)
(74) Mandataire: Neyret, Daniel Jean Marie
(86) Numéro de dépôt international: PCT/FR2004/001410
(87) Numéro de publication internationale: WO 2004/110318

(56) Documents cités:
- EP-A- 0 297 789
- WO-A-01/24739
- US-A- 5 080 677
- US-A- 5 645 594

## Description

L'invention concerne le domaine des prothèses articulaires, telles que les prothèses de hanche ou d'épaule.

Il est classique d'utiliser des prothèses de hanche composées, d'une part, d'une tige métallique et d'une tête fémorale en céramique de forme sensiblement sphérique que le chirurgien substitue à la partie supérieure du fémur du patient, et, d'autre part, d'un implant cotyloïdien destiné à recevoir ledit col fémoral, et que le chirurgien implante, par exemple par scellage, dans le bassin du patient à l'emplacement du cotyle naturel.

Dans un exemple connu d'une telle prothèse, l'implant cotyloïdien se compose de deux pièces :
- une cupule métallique fixée dans le bassin à l'emplacement du cotyle naturel au moyen de ciment ou de vis ;
- et un insert venant garnir l'intérieur de la cupule, destiné à recevoir la tête fémorale de la prothèse et présentant une forme correspondant à celle de la tête fémorale.

L'insert est, de préférence, réalisé en céramique de façon à présenter la meilleure résistance à l'usure lors des mouvements de la tête fémorale dans l'implant cotyloïdien. Mais ces inserts céramiques sont relativement coûteux. On peut donc souhaiter réserver leur emploi au cas de patients jeunes. Pour les patients plus âgés, on peut préférer utiliser des inserts en polyéthylène, certes moins résistants à l'usure, mais meilleur marché.

Cette dernière solution n'est cependant pas satisfaisante. L'assemblage entre la cupule métallique et l'insert en polyéthylène s'effectue par impaction, la coque présentant une portée conique, associée à différents moyens de fixation. Mais un maintien suffisamment ferme de l'insert dans la cupule n'est pas obtenu. Il subsiste des possibilités de micro-mouvements entre les deux pièces. Ces micro-mouvements provoquent le détachement de particules de polyéthylène qui pénètrent dans les trous de vis et viennent au contact de l'os. Cela engendre des ostéolyses et, à la longue, le descellement du cotyle par dégradation de la surface de contact os-métal.

Le but de l'invention est de proposer une nouvelle configuration d'implant cotyloïdien pour prothèse totale de hanche, autorisant l'utilisation d'un insert en polyéthylène associé à une cupule métallique, sans les inconvénients précités.

A cet effet, l'invention a pour objet un insert pour cupule d'implant cotyloïdien pour prothèse articulaire, du type comprenant une coque métallique et un garnissage en polymère de l'espace intérieur de ladite coque, un réceptacle pour une tête prothétique étant ménagé sur le garnissage, ladite coque présentant, sur son espace intérieur, des moyens pour éviter une extraction dudit garnissage hors de la coque et des moyens pour éviter une rotation du garnissage dans la coque, caractérisé en ce que lesdits moyens pour éviter une extraction du garnissage comportent un plot disposé sur le fond de la coque et présentant au moins une rainure ou une lèvre.

Lesdits moyens pour éviter une extraction dudit garnissage peuvent comporter au moins une lèvre.

Lesdits moyens pour éviter une extraction dudit garnissage peuvent comporter au moins une rainure.

Lesdits moyens pour éviter une rotation du garnissage peuvent comporter des bossages ménagés sur la surface de la paroi délimitant l'espace intérieur de la coque.

Lesdits bossages peuvent être des nervures s'étendant sur toute ou partie de la hauteur de l'espace intérieur de la coque.

Lesdits moyens pour éviter une rotation du garnissage peuvent comporter des dépressions ménagées sur la surface de la paroi délimitant l'espace intérieur de la coque.

Des bossages peuvent être ménagés sur la lèvre.

Des dépressions peuvent être ménagées sur la lèvre.

La partie antérieure de ladite coque peut présenter une conicité.

La paroi du réceptacle peut être revêtue de céramique.

Ledit garnissage peut être obtenu par une opération de thermocompression dudit polymère dans ledit espace intérieur de la coque suivie par un usinage.

Ledit garnissage peut être obtenu par une mise en forme préalable et être mis en place par impaction dans la coque.

L'invention a également pour objet un implant cotyloïdien du type comprenant une cupule destinée à être fixée dans le bassin ou l'omoplate d'un patient et un insert garnissant l'intérieur de ladite cupule pour recevoir une tête prothétique, caractérisé en ce que l'insert est du type précédent.

L'invention a également pour objet une prothèse articulaire du type comportant un implant cotyloïdien et une tête prothétique, caractérisée en ce que ledit implant est du type précédent.

Comme on l'aura compris, l'invention consiste d'abord à prévoir un insert comportant deux parties :
- une coque métallique de base ;
- et une partie en polyéthylène.

Des moyens d'ancrage tels que des lèvres et/ou des rainures périphériques et transversales, ménagées dans la coque métallique permettent d'assurer une immobilité totale du polyéthylène dans le métal. On évite ainsi les frottements susceptibles de provoquer un relargage de particules de polyéthylène dans l'environnement de l'insert.

Selon, l'invention, un élément essentiel de ces moyens d'ancrage est un plot disposé dans le fond de la coque, et présentant une rainure ou une lèvre.

L'invention sera mieux comprise à la lecture de la description qui suit, donnée en référence aux figures annexées suivante :

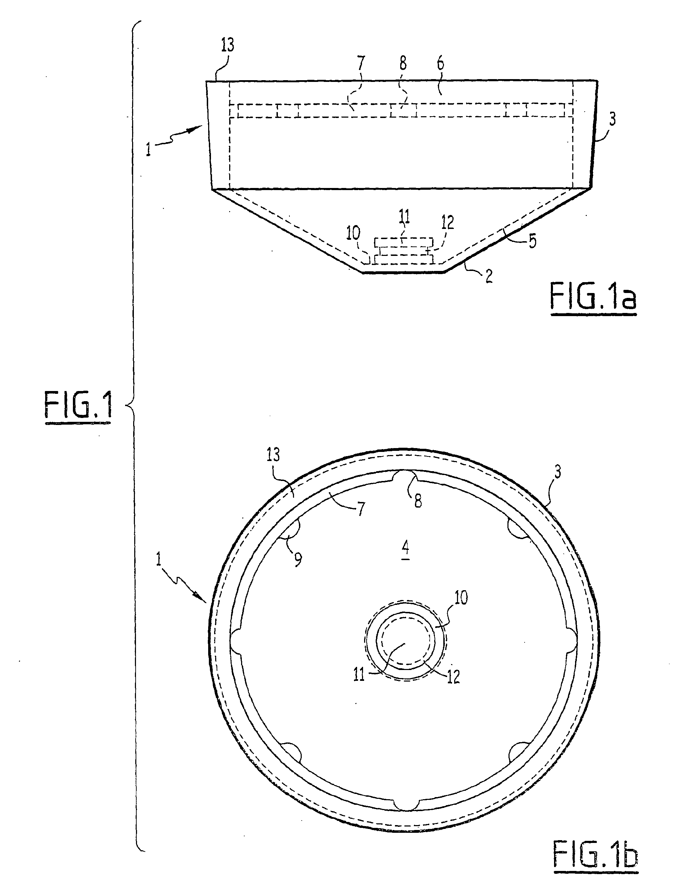

- la figure 1 qui montre vu de face (fig.1a) et de dessus (fig.1b) un exemple de coque métallique faisant partie d'un insert pour implant cotyloïdien selon l'invention ;
- la figure 2 qui montre schématiquement et séparément, en perspective et en vue partiellement arrachée, les deux éléments métallique et polymérique d'un exemple d'insert pour implant cotyloïdien selon l'invention.

La coque métallique (réalisée généralement en acier inoxydable ou en titane) 1 présente une forme extérieure tronconique (dans l'exemple représenté) ou sphérique dans sa partie postérieure 2, et une forme sensiblement cylindrique dans sa partie antérieure 3. De préférence, comme représenté, cette partie antérieure 3 présente une légère conicité qui, lorsque la cupule est en place dans le bassin ou l'omoplate du patient, contribue au maintien en place de la coque 1. Cette coque 1 est destinée à être mise en place dans un cotyle métallique (non représenté), lui-même fixé dans le bassin ou l'omoplate du patient par scellage ou vissage.

L'espace intérieur 4 de la coque 1, dans l'exemple représenté, est de forme tronconique dans sa partie postérieure 5, et de forme cylindrique dans sa partie antérieure 6. Ces formes pourraient, en variante, être respectivement sphérique (par exemple) et tronconique.

Le pourtour de la partie antérieure 6 de l'espace intérieur 4 de la coque 1 présente une lèvre en relief 7, elle-même pourvue de dépressions 8 et de bossages 9. Par ailleurs, selon l'invention, le fond 10 de la partie postérieure 5 de l'espace intérieur 4 de la coque 1 présente un plot 11 de forme sensiblement cylindrique, dont la paroi latérale est pourvue d'une rainure 12, comme représenté, et/ou d'une lèvre en relief, ou de plusieurs telles rainures 12 et/ou lèvres.

Selon un mode de réalisation, pour obtenir l'insert pour implant cotyloïdien selon l'invention, on commence par fabriquer une coque métallique du type de celle qui vient d'être décrite. Puis on remplit son espace intérieur par du polyéthylène (ou un autre polymère susceptible de convenir à ce type d'implants) par un procédé de thermocompression, c'est à dire d'injection à chaud sous pression. On fait également déborder le polyéthylène au-delà du bord supérieur 13 de la coque 1. Puis on usine le garnissage 14 en polyéthylène ainsi obtenu de façon à lui conférer une forme qui lui permettra de recevoir la tête de la prothèse de hanche ou d'épaule dont l'implant cotyloïdien selon l'invention fera partie.

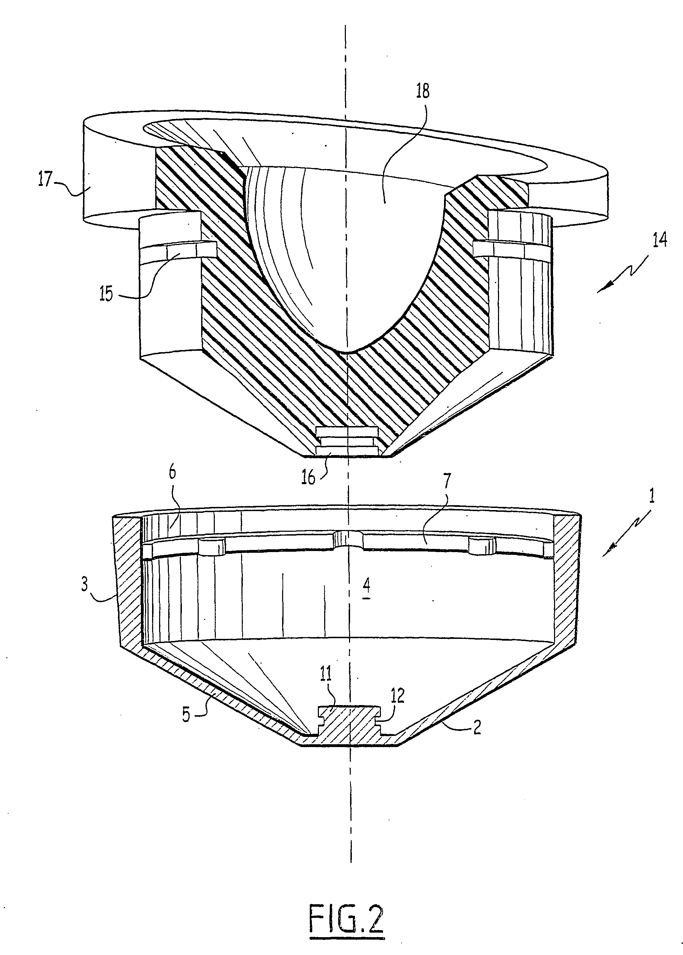

La figure 2 représente la coque 1 et son garnissage 14 en polyéthylène après usinage. Il doit être compris que ces deux éléments ne sont représentés séparément que pour la commodité de la représentation et que, en réalité, dans ce mode de réalisation le garnissage 14 demeure en permanence dans la coque 1. La lèvre 7 en relief a précisément pour fonction d'empêcher l'extraction du garnissage 14 après sa réalisation, en particulier lors de l'utilisation de l'implant selon l'invention. En effet, au cours de l'opération de thermocompression, il se forme une rainure 15 sur la périphérie extérieure du garnissage 14, au droit de la lèvre 7. La lèvre 7 pénètre donc dans le garnissage 14 et empêche tout mouvement du garnissage 14 qui tendrait à l'extraire de la coque 1.

Le plot 11 possède une fonction analogue. Lors de la thermocompression, le polyéthylène vient se mouler autour de lui, et la présence de la rainure 12 (respectivement d'une lèvre) procure une possibilité d'accrochage du garnissage 14 sur le plot 11, par formation sur le garnissage 14 d'une lèvre en relief 16 (respectivement d'une rainure) au droit de la rainure 12 (respectivement de la lèvre) du plot 11. La présence de ce plot 11 procure une résistance entièrement efficace à l'extraction du garnissage 14 et complète l'effet des autres éléments anti-extraction. Il peut même éventuellement constituer le seul élément anti-extraction

Quant aux mouvements de rotation du garnissage 14 à l'intérieur de la coque 1, ils sont empêchés par les dépressions 8 et les bossages 9 ménagés sur la lèvre 7.

De cette façon, les mouvements du garnissage 14 dans la coque 1 sont bloqués avec une efficacité optimale dans toutes les directions de l'espace. On évite ainsi que de tels mouvements ne provoquent un déplacement et une érosion du garnissage 14.

La configuration qui a été décrite et représentée pour les moyens autres que le plot 11 destinés à empêcher les déplacements du garnissage 14 dans la coque 1 et hors de la coque 1 n'est qu'un exemple.

La lèvre 7 peut être continue sur toute la périphérie de l'espace intérieur 4 de la coque 1, ou être discontinue. Il peut y en avoir plusieurs, réparties à différents niveaux de l'espace intérieur 4 de la coque 1. Elle pourrait être remplacée par une ou plusieurs rainures qui créeraient un relief correspondant sur le garnissage 14 lors de la thermocompression. Toutefois la lèvre 7 est la configuration préférée pour les moyens anti-extraction autres que le plot 11. En effet le polyéthylène, après la thermocompression, subit un retrait de 1,8%. Ce retrait tend à renforcer l'accrochage du garnissage 14 sur la coque 1 si celle-ci présente une lèvre 7, alors qu'une rainure tendrait à dégrader cet accrochage. Bien entendu, lèvres et rainures peuvent être combinées. Cet effet de renforcement de l'accrochage par le retrait du polyéthylène est également très sensible au niveau du plot 11, et, justement, la forme du plot 11 permet de tirer parti de cet effet de manière optimale.

En ce qui concerne les moyens anti-rotation, on peut ne prévoir que des dépressions 8, ou que des bossages 9, et les ménager à d'autres endroits que la lèvre 7 sur la surface de la paroi délimitant l'espace intérieur 4 de la coque 1. Pour la même raison que précédemment, liée au retrait du polyéthylène, les bossages 9 sont privilégiés. Ils peuvent avoir la forme de nervures s'étendant sur tout ou partie de la hauteur de l'espace intérieur 4 de la coque 1.

Comme on le voit sur la figure 2, l'usinage du garnissage 14 après la thermocompression permet de ménager d'une part un rebord 17 reposant sur le bord supérieur 13 de la coque 1, et surtout un réceptacle 18 de forme sphérique destiné à recevoir des têtes de différentes matières (céramiques ou métalliques) de la prothèse de hanche ou d'épaule à laquelle l'implant cotyloïdien selon l'invention est destiné à être intégré.

En variante on peut également incorporer, notamment par thermocompression, un revêtement céramique dans le réceptacle 18. De cette façon le contact entre l'insert et la tête de la prothèse, si celle-ci est en céramique, est un contact céramique-céramique qui évite tout relargage de polyéthylène dans l'environnement de la prothèse.

Dans un autre mode de réalisation, le garnissage 14 est réalisé non par thermocompression dans la coque 1, mais par une opération de mise en forme (moulage et/ou usinage par exemple) effectuée en dehors de la coque 1. La mise en place du garnissage 14 dans la coque 1 est ensuite effectuée par impaction dans la coque 1, le garnissage 14 présentant des creux et reliefs complémentaires des reliefs et creux présents sur l'espace intérieur 4 de la coque 1. Eventuellement, un dernier usinage du garnissage 14 après sa mise en place permet, si nécessaire, d'en ajuster les dimensions extérieures. Là encore, un revêtement céramique peut être ajouté dans le réceptacle 18.

## Revendications

1. Insert pour cupule d'implant cotyloïdien pour prothèse articulaire, du type comprenant une coque (1) métallique et un garnissage (14) en polymère de l'espace intérieur (4) de ladite coque (1), un réceptacle (18) pour une tête prothétique étant ménagé sur le garnissage (14), ladite coque présentant, sur son espace intérieur (4), des moyens pour éviter une extraction dudit garnissage (14) hors de la coque (1) et des moyens pour éviter une rotation du garnissage (14) dans la coque (1), **caractérisé en ce que** lesdits moyens pour éviter une extraction du garnissage comportent un plot (11) disposé sur le fond (10) de la coque (1) et présentant au moins une rainure (12) ou une lèvre.

2. Insert selon la revendication 1, **caractérisé en ce que** lesdits moyens pour éviter une extraction dudit garnissage (14) comportent au moins une lèvre (7).

3. Insert selon la revendication 1 ou 2, **caractérisé en ce que** lesdits moyens pour éviter une extraction dudit garnissage (14) comportent au moins une rainure.

4. Insert selon l'une des revendications 1 à 3, **caractérisé en ce que** lesdits moyens pour éviter une rotation du garnissage (14) comportent des bossages (9) ménagés sur la surface de la paroi délimitant l'espace intérieur (4) de la coque (1).

5. Insert selon la revendication 4, **caractérisé en ce que** lesdits bossages (9) sont des nervures s'étendant sur toute ou partie de la hauteur de l'espace intérieur (4) de la coque (1).

6. Insert selon l'une des revendications 1 à 5, **caractérisé en ce que** lesdits moyens pour éviter une rotation du garnissage (14) comportent des dépressions (8) ménagées sur la surface de la paroi délimitant l'espace intérieur (4) de la coque (1).

7. Insert selon les revendications 2 et 4 prises ensemble, **caractérisé en ce que** des bossages (9) sont ménagés sur la lèvre (7).

8. Insert selon les revendications 2 et 6 prises ensembles, **caractérisé en ce que** des dépressions (8) sont ménagées sur la lèvre (7).

9. Insert selon l'une des revendications 1 à 8, **caractérisé en ce que** la partie antérieure de ladite coque (1) présente une conicité.

10. Insert selon l'une des revendications 1 à 9, **caractérisé en ce que** la paroi du réceptacle (18) est revêtue de céramique.

11. Insert selon l'une des revendications 1 à 10, **caractérisé en ce que** ledit garnissage (14) est obtenu par une opération de thermocompression dudit polymère dans ledit espace intérieur (4) de la coque (1) suivie par un usinage.

12. Insert selon l'une des revendications 1 à 10, **caractérisé en ce que** ledit garnissage (14) est obtenu par une mise en forme préalable et **en ce qu'**il est mis en place par impaction dans la coque (1).

13. Implant cotyloïdien du type comprenant une cupule destinée à être fixée dans le bassin ou l'omoplate d'un patient et un insert garnissant l'intérieur de ladite cupule pour recevoir une tête prothétique, **caractérisé en ce que** l'insert est du type selon l'une des revendications 1 à 12.

14. Prothèse articulaire du type comportant un implant cotyloïdien et une tête prothétique, **caractérisée en ce que** ledit implant est du type selon la revendication 13.

## Claims

1. Insert for a cotyloid implant cup for a joint prosthesis, of the type comprising a metal shell (1) and a polymer liner (14) lining the inner space (4) of said shell (1), a receptacle (18) for a prosthetic head being formed on the liner (14), said shell having, in its inner space (4), means for avoiding extraction of said liner (14) from the shell (1) and means for avoiding rotation of the liner (14) in the shell (1), **characterized in that** said means for avoiding extraction of the liner comprise a stud (11) arranged on the bottom (10) of the shell (1) and having at least one groove (12) or a lip.

2. Insert according to Claim 1, **characterized in that** said means for avoiding extraction of said liner (14) comprise at least one lip (7).

3. Insert according to Claim 1 or 2, **characterized in that** said means for avoiding extraction of said liner (14) comprise at least one groove.

4. Insert according to one of Claims 1 to 3, **characterized in that** said means for avoiding rotation of the liner (14) comprise bosses (9) formed on the surface of the wall delimiting the inner space (4) of the shell (1).

5. Insert according to Claim 4, **characterized in that** said bosses (9) are ribs that run the entire height or part of the height of the inner space (4) of the shell (1).

6. Insert according to one of Claims 1 to 5, **characterized in that** said means for avoiding rotation of the liner (14) comprise depressions (8) formed on the surface of the wall delimiting the inner space (4) of the shell (1).

7. Insert according to Claims 2 and 4 in combination, **characterized in that** bosses (9) are formed on the lip (7).

8. Insert according to Claims 2 and 6 in combination, **characterized in that** depressions (8) are formed on the lip (7).

9. Insert according to one of Claims 1 to 8, **characterized in that** the anterior part of said shell (1) has a conicity.

10. Insert according to one of Claims 1 to 9, **characterized in that** the wall of the receptacle (18) is coated with ceramic.

11. Insert according to one of Claims 1 to 10, **characterized in that** said liner (14) is obtained by an operation involving thermocompression of said polymer in said inner space (4) of the shell (1), followed by machining.

12. Insert according to one of Claims 1 to 10, **characterized in that** said liner (14) is obtained by a preliminary shaping operation, and **in that** it is fitted in the shell (1) by impaction.

13. Cotyloid implant of the type comprising a cup intended to be fixed in the pelvis or scapula of a patient, and an insert lining the interior of said cup in order to receive a prosthetic head, **characterized in that** the insert is of the type according to one of Claims 1 to 12.

14. Joint prosthesis of the type comprising a cotyloid implant and a prosthetic head, **characterized in that** said implant is of the type according to Claim 13.

## Patentansprüche

1. Einsatz für Gelenkpfannen-Becherimplantat für Gelenkprothese des Typs, der eine Metallschale (1) und ein Polymer-Futter (14) für den Innenraum (4) der Schale (1) umfasst, wobei in dem Futter (14) ein Aufnahmeraum (18) für einen Prothesenkopf ausgebildet ist, wobei die Schale in ihrem Innenraum (4) Mittel, um ein Herausziehen des Futters (14) aus der Schale (1) zu verhindern, und Mittel, um eine Drehung des Futters (14) in der Schale (1) zu verhindern, umfasst, **dadurch gekennzeichnet, dass** die Mittel zum Verhindern eines Herausziehens des Futters einen Höcker (11) aufweisen, der auf dem Boden (10) der Schale (1) angeordnet ist und wenigstens eine Nut (12) oder eine Lippe aufweist.

2. Einsatz nach Anspruch 1, **dadurch gekennzeichnet, dass** die Mittel zum Verhindern eines Herausziehens des Futters (14) wenigstens eine Lippe (7) umfassen.

3. Einsatz nach Anspruch 1 oder 2, **dadurch gekennzeichnet, dass** die Mittel zum Verhindern eines Herausziehens des Futters (14) wenigstens eine Nut umfassen.

4. Einsatz nach einem der Ansprüche 1 bis 3, **dadurch gekennzeichnet, dass** die Mittel zum Verhindern einer Drehung des Futters (14) Vorsprünge (9) umfassen, die an der Oberfläche der den Innenraum (4) der Schale begrenzenden Wand ausgebildet sind.

5. Einsatz nach Anspruch 4, **dadurch gekennzeichnet, dass** die Vorsprünge (9) Rippen sind, die sich auf der gesamten Höhe des Innenraums (4) der Schale (1) oder auf einem Teil hiervon erstrecken.

6. Einsatz nach einem der Ansprüche 1 bis 5, **dadurch gekennzeichnet, dass** die Mittel zum Verhindern einer Drehung des Futters, (14) Vertiefungen (8) umfassen, die in der Oberfläche der den Innenraum (4) der Schale (1) begrenzenden Wand ausgebildet sind.

7. Einsatz nach den Ansprüchen 2 und 4 zusammengenommen, **dadurch gekennzeichnet, dass** die Vorsprünge (9) an der Lippe (7) ausgebildet sind.

8. Einsatz nach den Ansprüchen 2 und 6 zusammengenommen, **dadurch gekennzeichnet, dass** die Vertiefungen (8) in der Lippe (7) ausgebildet sind.

9. Einsatz nach einem der Ansprüche 1 bis 8, **dadurch gekennzeichnet, dass** der vordere Teil der Schale (1) eine Konizität aufweist.

10. Einsatz nach einem der Ansprüche 1 bis 9, **dadurch gekennzeichnet, dass** die Wand des Aufnahmeraums (18) mit einer Keramik beschichtet ist.

11. Einsatz nach einem der Ansprüche 1 bis 10, **dadurch gekennzeichnet, dass** das Futter (14) durch einen Wärmekompressionsvorgang des Polymers in dem Innenraum (4) der Schale (1), gefolgt von einer Bearbeitung, erhalten wird.

12. Einsatz nach einem der Ansprüche 1 bis 10, **dadurch gekennzeichnet, dass** das Futter (14) durch eine vorhergehende Formung erhalten wird und dass es durch Einschlagen in die Schale (1) angeordnet wird.

13. Gelenkpfannenimplantat des Typs, der einen Becher, der dazu bestimmt ist, im Becken oder im Schulterblatt eines Patienten befestigt zu werden, und einen Einsatz, mit dem der Innenraum des Bechers ausgefüttert ist, um einen Prothesenkopf aufzunehmen, umfasst, dass der Einsatz von dem Typ nach einem der Ansprüche 1 bis 12 ist.

14. Gelenkprothese des Typs, der ein Gelenkpfannenimplantat und einen Prothesenkopf umfasst, **dadurch gekennzeichnet, dass**, das Implantat von dem Typ nach Anspruch 13 ist.
